# EUROPEAN PATENT SPECIFICATION

(11) **EP 2 696 810 B1**
(45) Date of publication and mention of the grant of the patent: **05.04.2017**
(21) Application number: 12705766.9
(22) Date of filing: 16.02.2012
(51) Int. Cl.: A61F 2/34, A61F 2/30

(54) **ACETABULAR ROOF REINFORCEMENT PLATE**
VERSTÄRKUNGSPLATTE FÜR PFANNENDACH
PLAQUE DE RENFORT DE TOIT ACÉTABULAIRE

(30) Priority: 11.04.2011 US 201161473983 P
(43) Date of publication of application: 19.02.2014
(73) Proprietor: Resch, Herbert, 5026 Salzburg (AT)
(72) Inventor: FIERLBECK, Johann, A-5020 Salzburg (AT); NIEDERBERGER, Alfred, A-5020 Salzburg (AT); RESCH, Herbert, A-5026 Salzburg (AT)
(74) Representative: KLIMENT & HENHAPEL
(86) International application number: PCT/US2012/025423
(87) International publication number: WO 2012/141788

(56) References cited:
- EP-A1- 1 082 949
- WO-A1-2009/058969
- WO-A2-2007/118708
- FR-A1- 2 633 823
- US-A1- 2005 261 688
- US-A1- 2008 172 130

## Description

### Field of the Invention

The present invention relates to a device for treating a bone and, in particular, relates to an implant for fixing the acetabulum of a hip joint.

### Background

Fractures of the acetabulum often require surgical reconstruction using screws and plates to stabilize the fracture. In the field of revision hip replacement the acetabulum may be reinforced using an acetabular implant including a hemispherical basket with a plurality of flanges extending therefrom. The flanges must be manually bent to adjust the fit to the anatomy of the patient. Screws are inserted through holes in the flanges and the hemispherical basket in a non-angularly stable configuration to fix the acetabular implant to the bone. However, these osteosyntheses are generally unable to secure the fracture sufficiently to allow immediate weight bearing. Thus, the fractured acetabulum is often fixed in a first surgical procedure after which the patient is not allowed to place weight on the affected side of the pelvis for at least six weeks. During this time the patient is confined to the bed. After six weeks, an artificial hip may be implanted in a second surgical procedure after which weight may be placed on the joint. However, the pain and reduced mobility associated with these separate procedures have a significant negative impact on the health of patients who are often elderly. Among other problems, prolonged periods of immobility may negatively impact muscle volume, blood

### Summary of the Invention

The present invention is directed to an acetabular implant, as defined in claim 1.

### Brief Description of the Drawings

Fig. 1 shows a perspective view of an implant according to an exemplary embodiment of the present invention;
Fig. 2 shows another perspective view of the implant of Fig. 1; and
Fig. 3 shows a schematic drawing of the implant of Fig. 1.

### Detailed Description

The present invention may be further understood with reference to the following description and the appended drawings, wherein like elements are referred to with the same reference numerals. The present invention relates to a device for treating a bone and, in particular, relates to an implant for fixing the acetabulum of a hip joint. Exemplary embodiments of the present invention describe an acetabular implant configured to receive angularly stable bone fixation elements such that fractured portions of the acetabulum are securely fixed and immediate weight bearing thereon is possible.

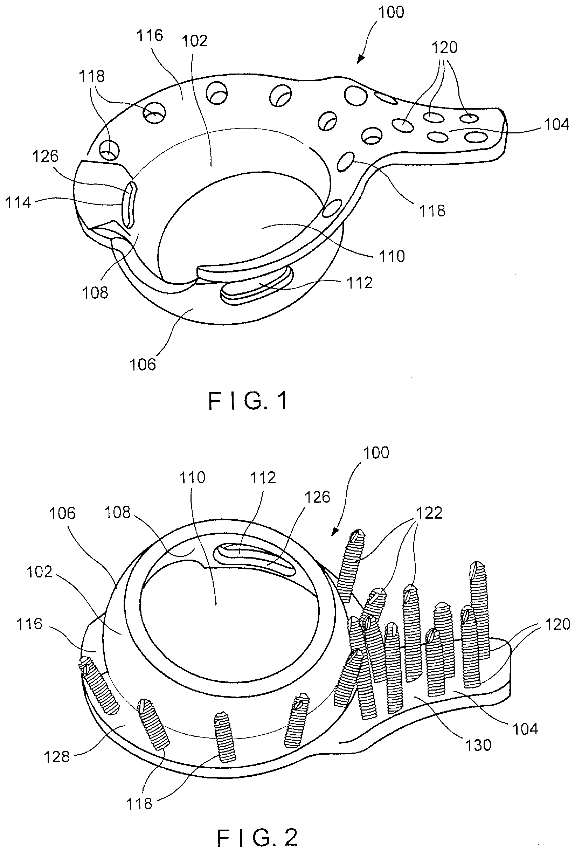

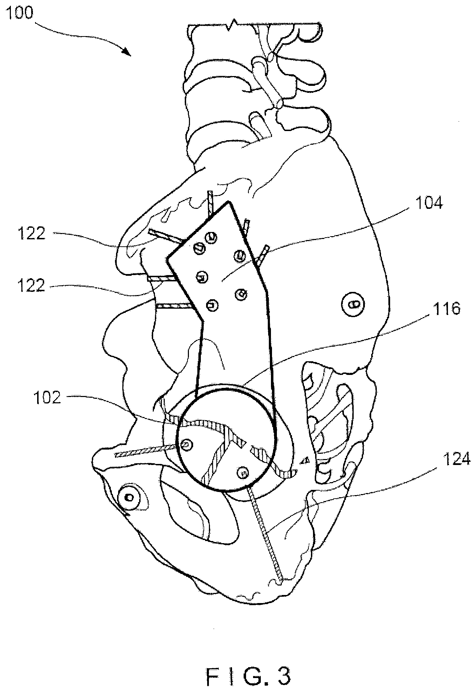

As shown in Figs. 1 - 3, an implant 100 according to an exemplary embodiment of the present invention comprises a body 102 and a flange 104 extending therefrom. The implant 100 according to this embodiment is configured to fix fractures of the acetabulum in a hip joint replacement and the body 102 is sized and shaped to engage the acetabulum while the flange 104 extends therefrom to engage the gluteal surface of the ilium (facies glutea). The implant 100 may be formed of a biocompatible metal such as, for example, titanium or stainless steel. It will be understood by those of skill in the art, however, that the implant 100 may be formed of any of a variety of biocompatible materials so long as the material possesses the required strength. In particular, the body 102 includes a substantially convex exterior surface 106 for engaging the acetabulum and a substantially concave interior surface 108 sized and shaped to receive a femoral head prosthesis. The substantially concave interior surface 108 may also be configured for attachment to an acetabular cup including a smooth surface for interfacing with the femoral head prosthesis during articulation. The implant 100 includes a rim 116 extending about at least a portion of a perimeter of the body 102 sized and shaped to engage an edge of the acetabulum. A plurality of bone fixation element receiving holes 118, 120, each configured to receive a bone fixation element 122 in an angularly stable configuration, are distributed in the flange 104 and the rim 116. The angularly stable connections between the bone fixation elements and the implant 100 permits immediate weight bearing so that the acetabular cup and/or the hip prosthesis (e.g., artificial femur head) may be implanted in a single procedure during which the implant 100 is also implanted.

The body 102 according to this embodiment is partially hemispherical and includes an opening 110 extending therethrough along a central axis of the body 102. As would be understood by those skilled in the art, bodies 102 may be provided in a variety of sizes to accommodate anatomical differences from patient to patient. For example, the body 102 may have an inner radius ranging from between 35 mm to 60 mm. The body 102 includes a first elongated compression hole 112 extending therethrough from the interior surface 108 to the exterior surface 106. The first compression hole 112 extends through a portion of the body 102 such that a shaft of a bone fixation element inserted therethrough extends through the acetabulum and into the pubis portion of the pelvis. The body 102 may also include a second elongated compression hole 114 extending therethrough from the interior surface 108 to the exterior surface 106 such that a shaft of a bone fixation element inserted therethrough may extend through the acetabulum into the ischium bone. Each of the compression holes 112, 114 includes a sloped, concave engagement surface 126 for engaging a spherical head of a bone fixation element 124. As would be understood by those skilled in the art, the spherical heads of bone fixation elements interface with the sloped surfaces 126 of the first and/or second compression holes 112, 114 to apply compression across a fracture. The partially hemispherical body 102 may have a radius selected to be slightly smaller than a radius of the acetabulum to facilitate compression of the fractured portions. In this embodiment, the first and second compression holes 112, 114, the body 102 are the only bone fixation element receiving holes formed in the body 102.

The rim 116 extends about at least a portion of a perimeter of the body 102 and is shaped to engage an edge or rim of the acetabulum. For example, the rim 116 may be curved, extending laterally from an edge of the body 102. The rim 116 includes a plurality of bone fixation element receiving holes 118. In accordance with the invention, each of the bone fixation element receiving holes 118 is configured to receive a bone fixation element therethrough in an angularly stable configuration along an axis thereof. However, as would be understood by those skilled in the art, one or more of these holes 118 may be a variable angle hole permitting a user to mount a bone fixation element therethrough at a user selected angle relative to an axis of the hole 118. The axes of one or more of the bone fixation element receiving holes 118 is angled with respect to an axis perpendicular to a bone contacting surface 128 adjacent the hole 118 such that a bone fixation element 122 inserted therethrough is angled with respect to the perpendicular axis. Several factors may determine the angle of the axes of the bone fixation element receiving holes 118 with respect to the perpendicular axis. For example, angles of the axes of the bone fixation element receiving holes should be selected such that the bone fixation element 122 inserted therein engages as much volume as possible in the thin bone while facilitating insertion through an existing surgical access path (e.g, the incision through which the implant was inserted). Those skilled in the art will understand that, although a surgical incision may extend along a surgical axis, surgical tools (e.g., drills, sleeves) and other items may be inserted therethrough angled relative to the surgical axis. Bone fixation elements 122 may thus be inserted into the holes 118 at any angle relative to the surgical access axis permitted by the surrounding tissue. In addition, angles of each of the axes may vary from one another for optimal anchorage in the bone. The holes 118 may, for example, including threading along inner surfaces thereof for lockingly engaging a threaded head of the bone fixation element 122 inserted thereinto. Thus, once the bone fixation elements 122 have been inserted through the holes 118 and engaged therewith, the bone fixation elements 122 are immovable relative to the implant 100.

The flange 104 extends radially from the body 102 and is sized and shaped to engage the gluteal surface of the ilium. In particular, the flange 104 may extend from a portion of the rim 116. Similarly to the rim 116, the flange 104 includes a plurality of bone fixation element receiving holes 120, each of which are configured to receive a bone fixation element 122 therethrough in an angularly stable configuration along an axis thereof. The angles of the axes of the holes 120 may be selected similarly to the axes of the holes 118, permitting bone fixation elements 122 to be inserted therethrough via the surgical site while providing optimal anchorage in the bone. The plurality of bone fixation element receiving holes 120 may include a threading extending along an inner surface thereof for engaging a threaded head of the bone fixation elements 122. Thus, similarly to the bone fixation element receiving holes 120 in the rim 116, once the bone fixation elements 122 are inserted into and engaged with the holes 120, the bone fixation elements 122 are immovable with respect to the implant 100.

As described above, the implant 100 is configured to treat fractures of the acetabulum. According to an exemplary surgical technique using the implant 100, the body 102 is positioned in the acetabulum with an exterior convex surface 106 thereof contacting the acetabulum while the flange 104 is positioned along the iliac of the pelvis. The rim 116 is positioned around an edge of the acetabulum. Bone fixation elements 122 (e.g., bone screws) are inserted through the bone fixation element receiving holes 118 along the rim 116 and the bone fixation element receiving openings 120 along the flange in an angularly stable configuration along axes thereof. Bone fixation elements 124 may also be inserted through the first and/or second compression holes 112, 114 to provide compression to the fractured portions of the bone as would be understood by those skilled in the art.

As described above, the angularly stable fixation of the implant via the bone fixation elements 122 provides sufficient strength of connection between the pelvis and the implant 100 that immediate weight bearing is possible. Thus, the patient is not required to wait six weeks for a follow-up procedure in which a hip replacement is to be performed. Rather, the acetabular cup and the prosthetic femur head may be implanted during the same surgical procedure. Once the implant 100 has been fixed to the pelvis, as desired, the acetabular cup may be attached to the interior surface 108 and a prosthetic femoral head may then be attached to the femur. The prosthetic femoral head and the acetabular cup are then able to interface with one another, providing articulation of the hip joint.

It will be apparent to those skilled in the art that various modifications and variations can be made in the structure and the methodology of the present invention, without departing from the scope of the invention. Thus, it is intended that the present invention cover the modifications and variations of this invention provided that they come within the scope of the appended claims.

## Claims

1. An acetabular implant (100), comprising:
a body (102) including a substantially convex exterior surface (106) configured to be received within and engage an acetabulum and a substantially concave interior surface (108) sized and shaped to receive a head portion of a femur and/or a femoral head implant, whereby said body (102) is designed partially hemispherical and includes an opening (110) extending therethrough along a central axis of the body (102);
a rim (116) extending about at least a portion of a periphery of the body (102) and including a plurality of first holes (118), each of the first holes (118) being configured to lockingly receive through an existing surgical access path, preferably through the incision through which the acetabular implant (100) was inserted, a bone fixation element (122) therethrough in an angularly stable configuration; and
a flange (104) extending radially from the rim (116) and including a plurality of second holes (120), each of the second holes (120) being configured to receive a bone fixation element (122) therethrough in an angularly stable configuration, **characterised in that** the axis of one or more of the first holes (118) is angled with respect to an axis perpendicular to a bone contacting surface (128) adjacent the hole (118) such that a bone fixation element (122) inserted therethrough is angled with respect to the perpendicular axis and whereby the axis of one or more of the first holes (118) is tilted towards said central axis of the body (102) and towards said surgical access path, such that the bone fixation elements (122) extend in a radial outward direction after insertion into the hole (118).

2. The implant of claim 1, further comprising an opening (110) extending through the body (102) along a central axis thereof.

3. The implant of claim 1 or 2, further comprising a first compression hole (112) extending through the body (102), the first compression hole (112) being elongated and including a sloped surface (126) therein configured to engage a spherical head of a bone fixation element (124), preferably further comprising a second compression hole (114) extending through the body (102), the second compression hole (114) being elongated and including a sloped surface (126) therein configured to engage a spherical head of a bone fixation element (124).

4. The implant of any one of claims 1 to 3, wherein the plurality of first holes (118) include a threading along an inner surface thereof configured to engage a threaded head of a bone fixation element (122).

5. The implant of any one of claims 1 to 4, wherein the plurality of second holes (120) include a threading along an inner surface thereof configured to engage a threaded head of a bone fixation element (122).

6. The implant of any one of claims 1 to 5, further comprising a variable angle hole extending through the rim (116), the variable angle hole configured to receive a bone fixation element therethrough at a user selected angle within a permitted range of angulation relative to a central axis thereof.

7. The implant of any one of claims 1 to 6, wherein the flange (104) is configured to engage an ilium portion of a pelvis.

8. The implant of claim 1, further comprising a first opening (110) defined by a portion of the cup-shaped body (102) adjacent to the rim (116) through which the one of a femoral head and a femoral head implant is inserted, and a second opening (110) extending through a portion of the body (102) opposite the first opening (110).

9. The implant of claim 1 or 8, further comprising a first compression hole (112) extending through the body (102), the first compression hole (112) being elongated and including a sloped surface (126) therein configured to slidingly engage a head of a bone fixation element (124) inserted therethrough to apply compression across a fracture of the acetabulum,
preferably further comprising a second compression hole (114) extending through the body (102), the second compression hole (114) being elongated and including a sloped surface (126) therein configured to slidingly engage a head of a bone fixation element (124) inserted therethrough to apply compression across a fracture of the acetabulum.

10. The implant of any one of claims 1 or 8 or 9, wherein the flange (104) is configured to engage a facies glutea.

## Patentansprüche

1. Hüftgelenkspfannenimplantat (100), umfassend:
einen Körper (102) mit einer im Wesentlichen konvexen Außenfläche (106), die dafür ausgelegt ist, in einer Hüftgelenkspfanne aufgenommen zu werden und in Eingriff mit dieser zu gelangen, und einer im Wesentlichen konkaven Innenfläche (108), die dafür bemessen und geformt ist,
einen Kopfabschnitt eines Oberschenkelknochens und/oder ein Oberschenkelkopfimplantat aufzunehmen, wobei der Körper (102) teilweise halbkugelförmig ausgestaltet ist und eine Öffnung (110) aufweist, die sich entlang einer Mittelachse des Körpers (102) durch ihn hindurch erstreckt;
einen Rand (116), der sich um wenigstens einen Abschnitt eines Umfangs des Körpers (102) erstreckt und eine Vielzahl von ersten Löchern (118) aufweist, wobei jedes der ersten Löcher (118) dafür ausgelegt ist, durch einen bestehenden chirurgischen Zugangsweg, vorzugsweise durch den Einschnitt, durch den das Hüftgelenkspfannenimplantat (100) eingeführt wurde, durch es hindurch ein Knochenfixierungselement (122) in einer winkelmäßig stabilen Konfiguration verriegelnd aufzunehmen; und
einen Flansch (104), der sich radial ausgehend vom Rand (116) erstreckt und eine Vielzahl von zweiten Löchern (120) aufweist, wobei jedes der zweiten Löcher (120) dafür ausgelegt ist, durch es hindurch ein Knochenfixierungselement (122) in einer winkelmäßig stabilen Konfiguration aufzunehmen,
**dadurch gekennzeichnet, dass**
die Achse von einem oder mehreren der ersten Löcher (118) in Bezug auf eine Achse, die senkrecht zu einer an das Loch (118) angrenzenden Knochenkontaktfläche (128) ist, derart abgewinkelt ist, dass ein Knochenfixierungselement (122), das durch es hindurch eingefügt ist, in Bezug auf die senkrechte Achse abgewinkelt ist, und wobei die Achse von einem oder mehreren der ersten Löcher (118) in Richtung auf die Mittelachse des Körpers (102) und in Richtung auf den chirurgischen Zugangsweg derart geneigt ist, dass sich die Knochenfixierungselemente (122) nach dem Einfügen in das Loch (118) in einer radial nach außen gerichteten Richtung erstrecken.

2. Implantat nach Anspruch 1, ferner umfassend eine Öffnung (110), die sich entlang einer Mittelachse von diesem durch den Körper (102) erstreckt.

3. Implantat nach Anspruch 1 oder 2, ferner umfassend ein erstes Kompressionsloch (112), das sich durch den Körper (102) erstreckt, wobei das erste Kompressionsloch (112) langgestreckt ist und eine geneigte Oberfläche (126) aufweist, die dafür ausgelegt ist, in Eingriff mit einem kugelförmigen Kopf eines Knochenfixierungselements (124) zu gelangen,
vorzugsweise ferner umfassend ein zweites Kompressionsloch (114), das sich durch den Körper (102) erstreckt, wobei das zweite Kompressionsloch (114) langgestreckt ist und eine geneigte Oberfläche (126) aufweist, die dafür ausgelegt ist, in Eingriff mit einem kugelförmigen Kopf eines Knochenfixierungselements (124) zu gelangen.

4. Implantat nach einem der Ansprüche 1 bis 3, wobei die Vielzahl von ersten Löchern (118) entlang ihrer Innenfläche ein Gewinde aufweist, das dafür ausgelegt ist, in Eingriff mit einem mit Gewinde versehenen Kopf eines Knochenfixierungselements (122) zu gelangen.

5. Implantat nach einem der Ansprüche 1 bis 4, wobei die Vielzahl von zweiten Löchern (120) entlang ihrer Innenfläche ein Gewinde umfasst, das dafür ausgelegt ist, in Eingriff mit einem mit Gewinde versehenen Kopf eines Knochenfixierungselements (122) zu gelangen.

6. Implantat nach einem der Ansprüche 1 bis 5, ferner umfassend ein Loch mit variablem Winkel, das sich durch den Rand (116) erstreckt, wobei das Loch mit variablem Winkel dafür ausgelegt ist, mit einem vom Benutzer gewählten Winkel innerhalb eines zulässigen Bereichs der Winkelung in Bezug auf seine Mittelachse ein Knochenfixierungselement durch es hindurch aufzunehmen.

7. Implantat nach einem der Ansprüche 1 bis 6, wobei der Flansch (104) dafür ausgelegt ist, in Eingriff mit einem Darmbeinabschnitt eines Beckens zu gelangen.

8. Implantat nach Anspruch 1, ferner umfassend eine erste Öffnung (110), die durch einen an den Rand (116) angrenzenden Abschnitt des schalenförmigen Körpers (102) definiert wird und durch die eines aus einem Oberschenkelkopf und einem Oberschenkelkopfimplantat eingefügt wird, und eine zweite Öffnung (110), die sich gegenüber der ersten Öffnung (110) durch einen Abschnitt des Körpers (102) erstreckt.

9. Implantat nach Anspruch 1 oder 8, ferner umfassend ein erstes Kompressionsloch (112), das sich durch den Körper (102) erstreckt, wobei das erste Kompressionsloch (112) langgestreckt ist und eine geneigte Oberfläche (126) aufweist, die dafür ausgelegt ist, gleitend in Eingriff mit einem Kopf eines Knochenfixierungselements (124) zu gelangen, das durch es hindurch eingefügt wird, um eine Kompressionswirkung auf einen Bruch der Hüftgelenkspfanne auszuüben,
vorzugsweise ferner umfassend ein zweites Kompressionsloch (114), das sich durch den Körper (102) erstreckt, wobei das zweite Kompressionsloch (114) langgestreckt ist und eine geneigte Oberfläche (126) aufweist, die dafür ausgelegt ist, gleitend in Eingriff mit einem Kopf eines Knochenfixierungselements (124) zu gelangen, das durch es hindurch eingefügt wird, um eine Kompressionswirkung auf einen Bruch der Hüftgelenkspfanne auszuüben.

10. Implantat nach einem der Ansprüche 1 oder 8 oder 9, wobei der Flansch (104) dafür ausgelegt ist, in Eingriff mit einer Außenfläche der Darmbeinschaufel zu gelangen.

## Revendications

1. Un implant acétabulaire (100) comprenant :
un corps (102) incluant une surface extérieure substantiellement convexe (106) configuré pour être reçu et pour s'engager dans la cavité cotyloïdienne et une surface intérieure substantiellement concave (108) dimensionnée et formée pour recevoir une portion de tête d'un fémur et/ou un implant de tête fémorale, où ledit corps (102) est conçu partiellement hémisphérique et inclut une ouverture (110) s'étendant au travers le long d'un axe central du corps (102) ;
un bord (116) s'étendant autour au moins d'une portion d'une périphérique du corps (102) et incluant une pluralité de premiers trous (118), chacun des premiers trous (118) étant configuré pour recevoir, de manière verrouillable, au travers d'un chemin d'accès chirurgical existant, de préférence par le biais de l'incision au travers de laquelle l'implant acétabulaire (100) a été inséré, un élément de fixation d'os (122) au travers dans une configuration angulairement stable ; et
une bride (104) s'étendant radialement à partir du bord (116) et incluant une pluralité de deuxièmes trous (120), chacun des deuxièmes trous (120) étant configuré pour recevoir un élément de fixation d'os (122) au travers dans une configuration angulairement stable, **caractérisée en ce que** l'axe d'un ou de plusieurs des premiers trous (118) est en angle par rapport à un axe perpendiculaire à une surface en contact avec un os (128) adjacente au trou (118) de sorte qu'un élément de fixation d'os (122) inséré au travers est en angle par rapport à l'axe perpendiculaire et où l'axe d'un ou de plusieurs des premiers trous (118) est incliné vers ledit axe central du corps (102) et vers ledit chemin d'accès chirurgical, de sorte que les éléments de fixation d'os (122) s'étendent dans un sens extérieur radial après l'insertion dans le trou (118).

2. L'implant de la revendication 1, comprenant de plus une ouverture (110) s'étendant au travers du corps (102) le long d'un axe central.

3. L'implant de la revendication 1 ou 2, comprenant de plus un premier trou de compression (112) s'étendant au travers du corps (102), le premier trou de compression (112) étant allongé et incluant une surface en pente (126) configurée pour engager une tête sphérique d'un élément de fixation d'os (124),
comprenant de plus, de préférence, un deuxième trou de compression (114) s'étendant au travers du corps (102), le deuxième trou de compression (114) étant allongé et incluant une surface en pente (126) configurée pour engager une tête sphérique d'un élément de fixation d'os (124).

4. L'implant de l'une des revendications 1 à 3, où la pluralité des premiers trous (118) inclut un filetage le long d'une surface intérieure configurée pour engager une tête filetée d'un élément de fixation d'os (122).

5. L'implant de l'une des revendications 1 à 4, où la pluralité des deuxièmes trous (120) inclut un filetage le long d'une surface intérieure configurée pour engager une tête filetée d'un élément de fixation d'os (122).

6. L'implant de l'une des revendications 1 à 5, comprenant de plus un trou d'angle variable s'étendant au travers du bord (116), le trou d'angle variable configuré pour recevoir un élément de fixation d'os au travers, à un angle sélectionné par l'utilisateur dans une fourchette permise d'angulation par rapport à un axe central.

7. L'implant de l'une des revendications 1 à 6, où la bride (104) est configurée pour engager une portion d'ilium d'un bassin.

8. L'implant de la revendication 1, comprenant de plus une première ouverture (110) définie par une portion du corps en forme de cupule (102) adjacente au bord (116) au travers de laquelle celle d'une tête fémorale et un implant de tête fémorale est inséré, et une deuxième ouverture (110) s'étendant au travers d'une portion du corps (102) à l'opposé de la première ouverture (110).

9. L'implant de la revendication 1 ou 8, comprenant de plus un premier trou de compression (112) s'étendant au travers du corps (102), le premier trou de compression (112) étant allongé et incluant une surface en pente (126) configurée pour engager de façon coulissante une tête d'un élément de fixation d'os (124) insérée au travers pour appliquer une compression sur une fracture de l'acétabulum,
comprenant de plus, de préférence, un deuxième trou de compression (114) s'étendant au travers du corps (102), le deuxième trou de compression (114) étant allongé et incluant une surface en pente (126) configurée pour engager, de façon coulissante, une tête d'un élément de fixation d'os (124) insérée au travers pour appliquer une compression sur une fracture de l'acétabulum.

10. L'implant de l'une des revendications 1 ou 8 ou 9, où la bride (104) est configurée pour engager un *facies glutea*.
